# EUROPEAN PATENT SPECIFICATION

(11) **EP 1 535 039 B1**
(45) Date of publication and mention of the grant of the patent: **06.10.2010**
(21) Application number: 03763396.3
(22) Date of filing: 10.07.2003
(51) Int. Cl.: G01N 3/00

(54) **STRAIN SENSING SYSTEM**
BELASTUNGSMESSSYSTEM
CAPTEUR EXTENSOMETRIQUE

(30) Priority: 10.07.2002 US 394607 P
(43) Date of publication of application: 01.06.2005
(73) Proprietor: OrthoData Inc., City of Wilmington, Delaware 19801 (US)
(72) Inventor: HNAT, William, P., Floyds Knobs, IN 47119 (US); NABER, John, F., Goshen, Kentucky 40026 (US); WALSH, Kevin, M., Louisville, KY 40207 (US)
(74) Representative: Schwabe - Sandmair - Marx
(86) International application number: PCT/US2003/021454
(87) International publication number: WO 2004/005872

(56) References cited:
- WO-A-00/56210
- WO-A-01/37726
- WO-A1-00/54237
- GB-A- 2 240 850
- US-A- 5 585 571
- US-A1- 2001 047 689
- US-A1- 2002 082 665
- US-A1- 2002 134 147
- US-B2- 6 529 127

## Description

### Field of the Invention

The present invention relates generally to a system for sensing and remotely monitoring strain in an element. More specifically, the present invention relates to a biomedical implant that incorporates a strain sensor and a telemetry circuit, and a remote reader module for measuring and monitoring strain in, for example, an orthopedic device located within a human or animal subject such that the resultant strain data can be analyzed to determine the progress of a healing injury or monitor the long term effectiveness of an implanted device.

### Background of the Invention

Many modern surgical techniques for the repair of damaged skeletal structure utilize implanted orthopedic devices affixed to the skeletal structure to lend support and rigidity thereto until the normal healing process progresses sufficiently that the structure is capable of its intended use. For example, spinal fusion surgery often involves implantation of a bio-compatible stainless steel or titanium spinal fusion implant comprised of a plurality of rods affixed to the damaged spine proximate the damaged area, usually by pedicle screws. The implant is designed to stabilize and support the spine until fusion occurs.

Presently there are several techniques available to a physician to monitor the healing or fusion process in an orthopedic implant. Common diagnostic tools include radiography, computer tomography (CT) and magnetic resonance imaging (MRI) scans, and of course exploratory surgery. Radiography, CT scans and MRI scans all are quite limited in their ability and accuracy in monitoring fusion progress due to the difficulty encountered in interpreting the scan results, even by experienced medical practitioners. Exploratory surgery is, of course, quite reliable for viewing fusion progress but is highly undesirable because of the various risks associated with an additional surgery. While some methods of measuring the progress of fusion in a patient presently exist, no known methods have the ability to monitor strain in an orthopedic device or other element (and thus the progress of the fusion taking place) under both static and dynamic loading conditions.

By carefully monitoring and quantifying the progress of spinal fusion, patients are able to return to normal activities sooner without risk of compromising the fusion process. The result is a reduction in doctor visits, decreased medical costs, and a reduction in lost work time and the attendant cost savings resulting therefrom. The average time for spinal fusion to occur is between 6 and 12 months. A real-time monitoring system for spinal fusion will eliminate the need for more costly procedures such as CT and MRI scans and provide surgeons with valuable information during the treatment process. Elimination of a single follow-up CT scan alone could save over $1000 per patient. Furthermore, fusion failures can be diagnosed more quickly and accurately thereby permitting the orthopedic surgeon to take corrective measures immediately when the fusion process is not progressing space.

Document WO 01/37726 discloses a medical implant device which includes a structure implantable within a body of a living animal for assisting in carrying out function within the body. Therefore a transducer element mounted on a carrier for sensing a parameter associated with the structure is coupled to a communicaton circuit for producing an output based on the sensed parameter and which serves to communicate the output non-invasively to a receiver located outside the body.

### Summary of the Invention

The present invention provides a miniature sensor for measuring strain as defined in claim 1. The telemetry circuit and sensor may be powered via inductive coupling from the reader so that no power source is required to be placed in vivo in implantation applications. Furthermore, a bio-compatible housing is used to encapsulate the sensor and telemetry components and provide a convenient method for mounting the system on orthopedic implant devices, as well as provide some measure of strain amplification.

Commercially available orthopedic devices such as spinal fixation rods can be equipped with the proposed monitoring system and used to measure the strain in the device, thereby providing the surgeon with a reliable and cost effective method to determine the success of the orthopedic implant in vivo. The monitoring system may also be used as a warning system for implant failures since the rod strains will necessarily decrease as healing progresses. Rod strain levels that do not decrease over time, increase somewhat, or change abruptly could be indicative of implant failure. The monitoring system may also be used with orthopedic screws, pins, plates, and joint implants.

The present invention provides a physician with the ability to monitor the spinal fusion process by measuring quantitatively the spinal fixation rod strains. The in vivo load transfer from the spinal fusion rod to the spine is monitored in real time using a miniature strain sensor placed indirectly on the surface of the rod. This data is then transmitted outside the body using the internal telemetry circuit and external reader, and evaluated instantaneously by the surgeon. In a successful fusion surgery, as the spine fuses the load on the spine is transferred from the rod to the spine, thereby lowering the monitored strain on the implant rod surface. The load transfer for a normal spinal fusion should be gradual and any deviation would indicate either non-fusion or possible failure of a rod or pedicle screw used to secure the rod to the spine.

It is therefore one object of the instant invention to provide a system for measuring and monitoring strain in an element.

A further object of the invention is a system that remotely monitors strain in a loaded element.

A further object of the invention is a system for measuring in vivo strain on an orthopedic device.

A further object of the invention is a system for measuring in vivo strain in an orthopedic device in real time.

A further object of the invention is an implantable, bio-compatible sensor and telemetry system for measuring in vivo strain.

Other uses, advantages, and features of the instant invention will become apparent after reading the detailed description of the preferred embodiments taken in conjunction with the accompanying drawing figures.

### Brief Description of the Drawing Figures

Fig. 1 is a block diagram of the strain measuring system in accordance with the present invention.

Fig. 2 is a block diagram of a capacitance sensor in accordance with the present invention.

Fig. 3 is a block diagram of the strain measuring system in accordance with the present invention.

Fig. 4 is an isometric view of a sensor housing in accordance with the present invention.

Fig. 5 is an isometric view of a sensor housing in accordance with the present invention.

Fig. 6 is a diagram of a spinal fusion orthopedic implant equipped with the present invention.

Fig. 7 is a block diagram of the system of the present invention.

### Detailed Description of the Preferred Embodiments

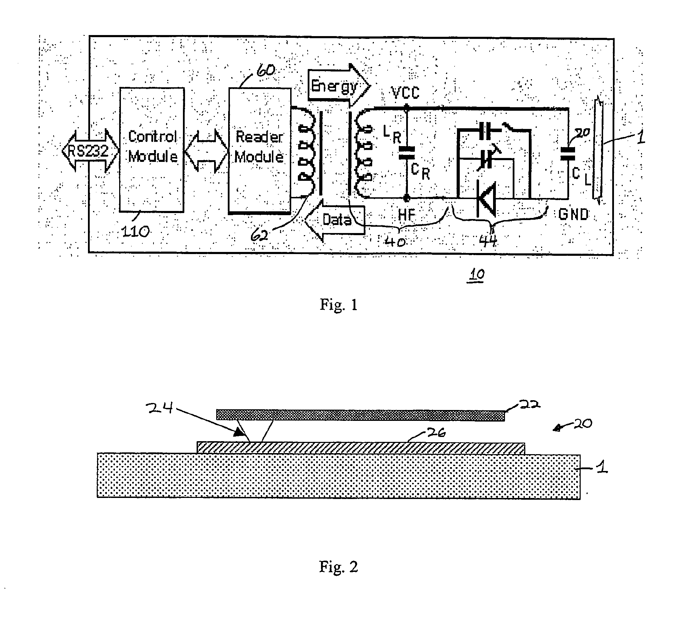

Referring to Fig. 1 and in accordance with one embodiment of the instant invention, a system 10 for measuring and remotely monitoring strain in an element 1 subject thereto includes a sensor 20 capable of measuring static and dynamic strain in the element 1, a telemetry circuit 40 that transmits sensor 20 data, and a remotely located reader module 60 for receiving the transmitted sensor data. The sensor 20 can be a miniaturized strain gauge, a MEMS (micro electrical mechanical system) sensor, a surface acoustic wave (SAW) sensor, or a capacitance-type sensor adapted to measure strain in an element, or any other strain sensor capable of measuring both static and dynamic strain in a loaded element 1. Each of the aforementioned sensors 20 consume relatively little electrical power and thus are advantageous for use in the instant system 20 when an in vivo application is necessary.

Referring to Fig. 2, a capacitance-type cantilevered beam sensor 20 may be employed with the present invention, wherein a capacitance beam 22 acting as a first parallel plate depends from a pivot 24 secured to a slipcover 26 that permits the sensor to be mounted on the strained element 1, or alternatively on a housing encapsulating the sensor 20, in addition to acting as the second parallel plate of the sensor 20. As the element 1 flexes, the distance between the beam 22 and slipcover 26 varies, thereby varying the capacitance of the sensor.

Referring again to Fig. 1, a passive telemetry circuit 40 is provided (requiring no battery) that includes an inductor L_{R} and capacitor C_{R} forming a simple tank circuit. The reader module 60 utilizes an antenna coil 62 that transmits at a predetermined frequency, for example 125 KHz, as is common in radio frequency identification device (RFID) circuitry. The power transmitted from the antenna 62 inductively couples the telemetry circuit 40, thereby causing it to resonate at a particular frequency depending upon the inductance and capacitance values.

As the capacitance of the strain sensor C_{L} varies with the strain as measured in element 1, the resonant frequency of the telemetry circuit 40 changes responsive to the strain. The reader 60 then detects the corresponding resonant frequency signal produced by the telemetry circuit 40 that is indicative of strain in the element 1.

In one embodiment of the invention, a simple power circuit 44 is included to provide rectified dc power derived from the power transmitted from the reader antenna 62 to the telemetry circuit 40 to be utilized to power additional circuitry such as signal processing (not shown) for the sensor 20 signal.

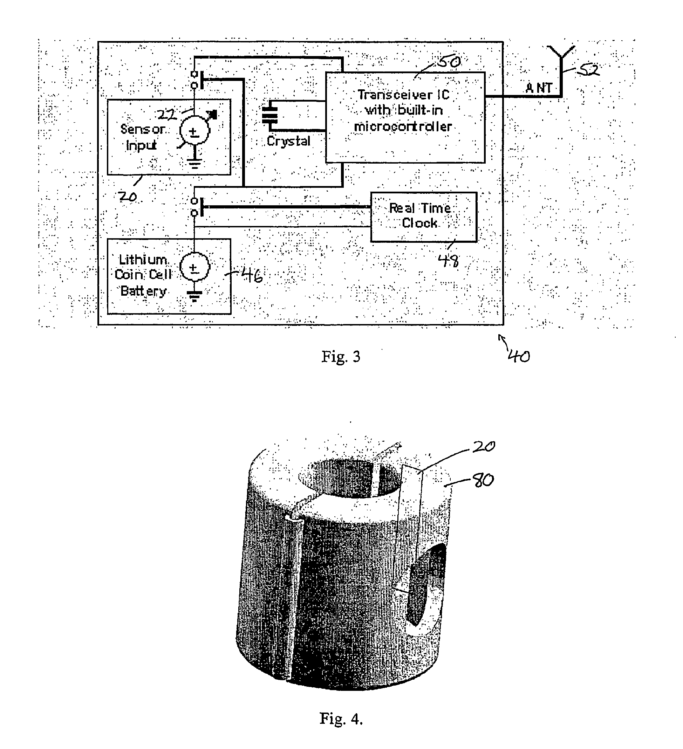

Referring to Fig. 3, an alternative telemetry circuit 40 is shown, whereby a miniature power supply 46, for example a lithium battery, is used to actively power the telemetry circuit 40. A real time clock 48 is used as a switch to energize and de-energize the entire circuit 40 at predetermined intervals in order to preserve battery 46 power. In this embodiment of the invention a transceiver integrated circuit (IC) 50 is used to accept the sensor 20 input 22 and transmit the input to the remote reader 60. This embodiment of the present invention permits the use of a conventional strain gauge as a sensor 20, since sufficient dc power is readily available from the battery 46, as well as an on-board microcontroller for processing and storing the data from the sensor 20. The sensor 20 data is then transmitted via radio frequency communication through an antenna 52. This embodiment of the present invention also permits the use of a variety of commercially available IC packages as a transceiver 50 for use in storing and transmitting the sensor 20 data.

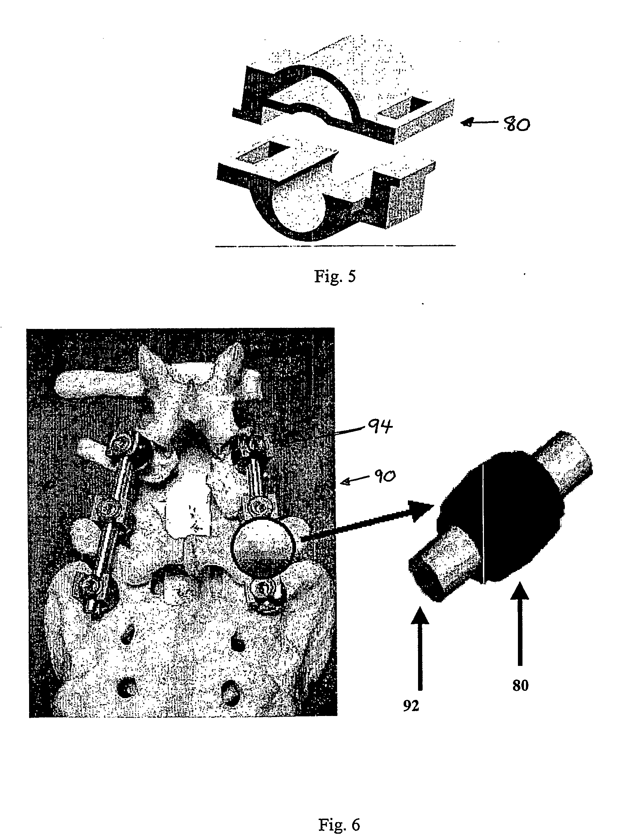

Figs. 4 and 5 depict two housings 80 that may be used to encapsulate the sensor 20 and telemetry circuit, and are advantageous for use in inter-vivo applications. These housings 80 are suitable for use where the sensor 20 is used to measure strain in a rod or similar device, for example as a component of an orthopedic implant. As one example of orthopedic use, Fig. 6 shows a spinal fusion implant 90 comprising a plurality of rods 92 affixed by a plurality of pedicle screws 94 both above and below a pair of vertebrae being fused. This orthopedic implant 90 is used to stabilize and support the surgically fused vertebrae until the healing process fuses the vertebrae sufficiently to bear the load required of the spine. Over time as the fused vertebrae heal, there is an in vivo load transfer from the implant 90 to the spine. Thus by monitoring the strain in the implant rods 92 over time, a physician can determine the progression of the spinal fusion.

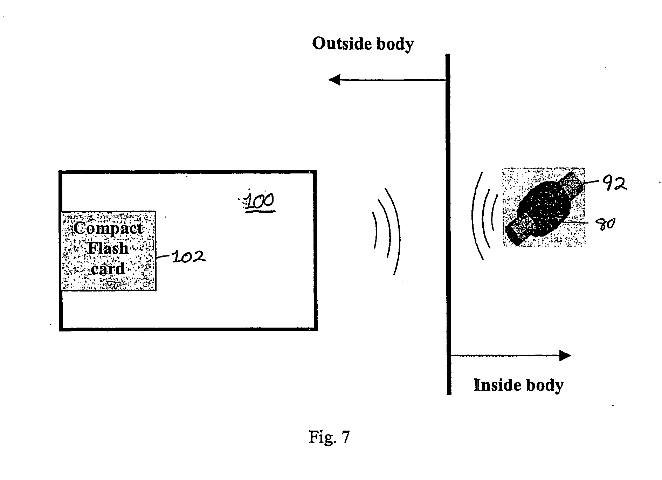

The housings 80 are made of any bio-compatible material such as polyethylene or a similar non-reactive polymer to permit the sensor 20 and telemetry circuit 40 encapsulated therein to be implanted in a living organism. As best seen in Figs. 6 and 7 the substantially annular housings 80 can be placed around the circumference of an implant rod 92 such that the sensor 20 is disposed on the rod 92 surface which is an example not according to the invention. Furthermore, the housings 80 comprises two interlocking halves to facilitate the placement of the telemetry circuit 40 and sensor 20 within the housing, and permit ease of installation of the entire assembly onto an implant rod. This feature of the invention permits a sensor 20 and concomitant telemetry circuit 40 to be affixed to the implant rod or rods 92 in advance of the surgery, thereby reducing operating time. While the specific housing embodiments shown in Figs. 4 and 5 are adapted to be used with cylindrical rods, it will be appreciated by one of ordinary skill that a variety of implant shapes can be accommodated by modification of the interior surface of the housing 80.

According to the invention, the sensor 20 is placed so that it does not directly contact the surface of the implant rod 90, but instead is in contact with an interior surface of the housing 80. As the rod 90 is strained, the housing 80 is also strained, thereby imparting strain to the sensor 20, and even amplifying the strain in the rod 90 to some extent.

In a further preferred embodiment of the invention a compact battery-powered reader 100 and an associated flash card memory 102 may be employed as a belt or pocket unit, similar to a conventional pager, that may be located on a belt or other location proximate to an implanted orthopedic device instrumented with the invention. The compact reader 100 provides sufficient power to the sensor 20 and telemetry circuit 40 to receive sensor 20 data at pre-determined intervals throughout the day whereupon it is stored in the memory 102. The flash memory card 102 may be removed from the reader 100 periodically, and the data stored thereon may be downloaded to a conventional computer (not shown) for use by a physician. This feature of the invention permits the physician to monitor in near real-time the progress of the fusion process, or other strain data indicative of the progress of orthopedic implant surgery. Furthermore, since the flash memory card 102 can be readily used to transmit the stored strain data to a conventional personal computer, the physician can have near real-time access to the data in event of an emergency or related concern from a recovering patient.

Additionally, a conventional microcomputer control module 110 may be employed in communication with the reader 60 to store and process the sensor 20 data and may be used to construct graphical representations of the strain data, or transmit the data to others.

## Claims

1. A system (10) for measuring and remotely monitoring strain in an orthopedic implant rod (92) comprising:
- a sensor (20) for measuring strain in said rod (92) producing an electrical signal representative thereof;
- a telemetry circuit (40) electrically coupled to said sensor (20) for encoding and transmitting the signal representative of strain;
- and a reader module (60) remotely located from said sensor (20) and said telemetry circuit (40) for receiving the signal representative of strain; and
- an annular housing (80) comprising a bio-compatible material;
**characterised in that**
- the sensor (20) and the telemetry circuit (40) are encapsulated in the housing (80) wherein the sensor (20) is placed so that it is in contact with an interior surface of the housing (80) and, if the housing (80) is placed around the circumference of the rod (92), it does not directly contact the surface of the rod (92), and **in that** the housing (80) comprises two interlocking halves which are placeable around the circumference of the implant rod (92) such that as the rod (92) is strained, the housing (80) is also strained, thereby imparting strain to the sensor (20).

2. The system (10) as claimed in claim 1 wherein said sensor (20) for measuring strain is a capacitive sensor.

3. The system (10) as claimed in claim 1 wherein said sensor (20) for measuring strain is a cantilever beam type capacitive sensor.

4. The system (10) as claimed in claim 1 wherein said sensor (20) for measuring strain is a surface acoustic wave sensor.

5. The system (10) as claimed in claim 1 wherein said sensor (20) for measuring strain is a miniaturized strain gauge.

6. The system (10) as claimed in claim 1 wherein said reader module (60) further comprises a concomitant removable memory card (102) for storing sensor data.

7. The system (10) as claimed in claim 6 wherein said reader module (60) is battery powered.

8. The system (10) according to claim 1 wherein by said reader module (60) power is transmittable to said telemetry circuit (40); the system (10) further comprising a control module (110) in communication with said reader module (60) for storing and processing the signal representative of strain.

## Patentansprüche

1. System (10) zum Messen und entfernten Überwachen der Beanspruchung in einem orthopädischen Implantatstab (92), das umfasst:
- einen Sensor (20) zum Messen der Beanspruchung in dem Stab (92), der ein hierfür repräsentatives elektrisches Signal erzeugt;
- eine Telemetrieschaltung (40), die mit dem Sensor (20) elektrisch gekoppelt ist, um das die Beanspruchung repräsentierende Signal zu codieren und zu senden;
- und ein Lesemodul (60), das sich entfernt von dem Sensor (20) und der Telemetrieschaltung (40) befindet, um das die Beanspruchung repräsentierende Signal zu empfangen; und
- ein ringförmiges Gehäuse (80), das ein biokompatibles Material enthält; **dadurch gekennzeichnet, dass**
- der Sensor (20) und die Telemetrieschaltung (40) in das Gehäuse (80) eingekapselt sind, wobei der Sensor (20) so angeordnet ist, dass er mit einer inneren Oberfläche des Gehäuses (80) in Kontakt ist und, falls das Gehäuse (80) um den Umfang des Stabs (92) angeordnet ist, mit der Oberfläche des Stabs (92) nicht direkt in Kontakt ist, und dass das Gehäuse (80) zwei aneinander befestigbare Hälften aufweist, die um den Umfang des Implantatstabs (92) angeordnet werden können, so dass, wenn der Stab (92) beansprucht wird, auch das Gehäuse (80) beansprucht wird, wodurch der Sensor (20) mit einer Beanspruchung beaufschlagt wird.

2. System (10) nach Anspruch 1, wobei der Sensor (20) zum Messen einer Beanspruchung ein kapazitiver Sensor ist.

3. System (10) nach Anspruch 1, wobei der Sensor (20) zum Messen einer Beanspruchung ein kapazitiver Sensor vom Typ einseitig unterstützter Träger ist.

4. System (10) nach Anspruch 1, wobei der Sensor (20) zum Messen einer Beanspruchung ein Oberflächenschallwellen-Sensor ist.

5. System (10) nach Anspruch 1, wobei der Sensor (20) zum Messen einer Beanspruchung ein miniaturisierter Dehnungsmessstreifen ist.

6. System (10) nach Anspruch 1, wobei das Lesemodul (60) ferner eine zugehörige entnehmbare Speicherkarte (120) zum Speichern von Sensordaten umfasst.

7. System (10) nach Anspruch 6, wobei das Lesemodul (60) durch eine Batterie mit Leistung versorgt wird.

8. System (10) nach Anspruch 1, wobei durch das Lesemodul (60) Leistung an die Telemetrieschaltung (40) übertragen werden kann; wobei das System (10) ferner ein Steuermodul (110) umfasst, das mit dem Lesemodul (60) kommunizieren kann, um das eine Beanspruchung repräsentierende Signal zu speichern und zu verarbeiten.

## Revendications

1. Système (10) pour mesurer et contrôler à distance une contrainte dans une tige d'implant orthopédique (92), comprenant :
- un capteur (20) pour mesurer une contrainte dans ladite tige (92), produisant un signal électrique représentatif de celle-ci ;
- un circuit de télémétrie (40) électriquement couplé audit capteur (20) pour coder et transmettre le signal représentatif de la contrainte ;
- et un module de lecteur (60) disposé à distance dudit capteur (20) et dudit circuit de télémétrie (40) pour recevoir le signal représentatif de la contrainte ; et
- un boîtier annulaire (80) comprenant un matériau bio-compatible ;
**caractérisé en ce que** :
- le capteur (20) et le circuit de télémétrie (40) sont encapsulés dans le boîtier (80), le capteur (20) étant disposé de façon à être en contact avec une surface intérieure du boîtier (80), et, si le boîtier (80) est disposé autour de la circonférence de la tige (92), il n'est pas directement en contact avec la surface de la tige (92), et **en ce que** le boîtier (80) comprend deux moitiés à verrouillage mutuel qui peuvent être disposées autour de la circonférence de la tige d'implant (92) de telle sorte que, lorsque la tige (92) est contrainte, le boîtier (80) soit également contraint, de façon à communiquer ainsi la contrainte au capteur (20).

2. Système (10) selon la revendication 1, dans lequel ledit capteur (20) pour mesurer une contrainte est un capteur capacitif.

3. Système (10) selon la revendication 1, dans lequel ledit capteur (20) pour mesurer une contrainte est un capteur capacitif du type à poutre en porte-à-faux.

4. Système (10) selon la revendication 1, dans lequel ledit capteur (20) pour mesurer une contrainte est un capteur d'onde acoustique de surface.

5. Système (10) selon la revendication 1, dans lequel ledit capteur (20) pour mesurer une contrainte est une jauge de contrainte miniaturisée.

6. Système (10) selon la revendication 1, dans lequel ledit module de lecteur (60) comprend de plus une carte mémoire amovible concomitante (102) pour mémoriser des données de capteur.

7. Système (10) selon la revendication 6, dans lequel ledit module de lecteur (60) est alimenté par pile ou batterie.

8. Système (10) selon la revendication 1, dans lequel, à l'aide dudit module de lecteur (60), du courant peut être transmis audit circuit de télémétrie (40) ; le système (10) comprenant de plus un module de commande (110) en communication avec ledit module de lecteur (60) pour mémoriser et traiter le signal représentatif de la contrainte.
